# EUROPÄISCHE PATENTANMELDUNG

(11) **EP 3 318 545 A1**
(43) Veröffentlichungstag der Anmeldung: **09.05.2018**
(21) Anmeldenummer: 16197156.9
(22) Anmeldetag: 03.11.2016
(51) Int. Cl.: C07C 5/48, C07C 11/04

(54) **VERFAHREN UND ANLAGE ZUR HERSTELLUNG VON OLEFINEN**

(71) Anmelder: Linde Aktiengesellschaft, 80331 München (DE)
(72) Erfinder: FRITZ, Helmut, 81375 München (DE); Zellhuber, Mathieu, 82152 Martinsried (DE); Wellenhofer, Anton, 82069 Hohenschäftlarn (DE); Schubert, Martin, 81375 München (DE); Winkler, Florian, 81371 München (DE)
(74) Vertreter: m patent group

(57) **Zusammenfassung**

Es wird ein Verfahren zur Herstellung eines oder mehrerer Olefine vorgeschlagen, bei dem ein Reaktionseinsatz gebildet wird, der ein oder mehrere Paraffine enthält, und bei dem ein Teil des oder der in dem Reaktionseinsatz enthaltenen Paraffine unter Erhalt eines Prozessgases durch oxidative Dehydrierung zu dem oder den Olefinen umgesetzt wird, wobei das Prozessgas zumindest das oder die Olefine, das oder die nicht umgesetzten Paraffine, Sauerstoff und Kohlenmonoxid enthält, und wobei zumindest ein Teil des Prozessgases einer Tieftemperaturtrennung unterworfen wird, in der auf einem Betriebsdruckniveau eine oder mehrere gegenüber dem Prozessgas an Sauerstoff und Kohlenmonoxid angereicherte Gasfraktionen gebildet werden. Es ist vorgesehen, dass in der Tieftemperaturtrennung bei der Bildung und/oder zur Führung der oder zumindest einer der Gasfraktionen ein oder mehrere Behälter und/oder eine oder mehrere Leitungen mit einem Berstdruck verwendet werden, der zumindest dem Zehnfachen des Betriebsdruckniveaus entspricht. Eine entsprechende Anlage (100) ist ebenfalls Gegenstand der vorliegenden Erfindung.

## Beschreibung

Die Erfindung betrifft ein Verfahren zur Herstellung von Olefinen und eine entsprechende Anlage gemäß den Oberbegriffen der unabhängigen Patentansprüche.

### Stand der Technik

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den Olefinen gleicher Kohlenstoffzahl und zu Wasser umgesetzt.

Die ODH kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen keine thermodynamische Gleichgewichtslimitierung. Die Bildungsenergien ΔG für Ethan, Propan bzw. n-Butan betragen -102, -115 bzw. -118 kJ/mol. Bei der ODH können vergleichsweise geringe Reaktionstemperaturen eingesetzt werden. Grundsätzlich ist keine Regenerierung der eingesetzten Katalysatoren erforderlich, da die Anwesenheit von Sauerstoff eine insitu-Regenerierung ermöglicht. Schließlich werden im Gegensatz zum Steamcracken geringere Mengen an wertlosen Nebenprodukten wie Koks gebildet.

Zu weiteren Details bezüglich der ODH sei auf einschlägige Fachliteratur, beispielsweise Ivars, F. und López Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, verwiesen.

Die Erfindung wird nachfolgend insbesondere unter Bezugnahme auf die ODH von Ethan (sogenannte ODH-E) beschrieben. Ihr Einsatz ist jedoch grundsätzlich auch bei der ODH höherer Paraffine wie Propan und Butan möglich und vorteilhaft.

Für die nachhaltige Aktivität der Katalysatoren in der ODH ist ein Mindestmaß an Sauerstoff am Reaktoraustritt erforderlich, um eine Reduktion des Katalysators und damit einen Verlust in dessen Leistungsfähigkeit zu vermeiden. Aus diesem Grund kann in der Regel nicht mit einem vollständigen Sauerstoffumsatz im Reaktor gearbeitet werden. Des Weiteren werden bei höheren Umsätzen nennenswerte Mengen an Kohlenmonoxid und Kohlendioxid als Nebenprodukte gebildet, die somit genauso wie der restliche Sauerstoff im Prozessgas aus dem Reaktor enthalten sind und in der ODH nachgeschalteten Trennschritten vom dem oder den Hauptprodukten und anderen Nebenprodukten abgetrennt werden müssen.

Wie auch nachfolgend erläutert, kann es dabei dazu kommen, dass sich in der Trennung tiefer siedender von höher siedenden Fraktionen insbesondere Kohlenmonoxid und Sauerstoff unerwünscht und über sicherheitstechnisch bedenkliche Konzentrationen hinaus anreichern.

Die vorliegende Erfindung stellt sich die Aufgabe, entsprechende Verfahren und Anlagen zu verbessern und insbesondere das Problem der Anreicherung von Kohlenmonoxid und Sauerstoff in einer entsprechenden Trennung in der ODH, insbesondere der ODH-E, zu adressieren.

### Offenbarung der Erfindung

Vor diesem Hintergrund schlägt die vorliegende Erfindung ein Verfahren zur Herstellung von Olefinen und eine entsprechende Anlage mit den Merkmalen der unabhängigen Patentansprüche vor. Ausgestaltungen sind jeweils Gegenstand der abhängigen Patentansprüche sowie der nachfolgenden Beschreibung.

Stoffströme, Gasgemische usw. können im hier verwendeten Sprachgebrauch reich oder arm an einer oder mehreren Komponenten sein, wobei die Angabe "reich" für einen Gehalt von wenigstens 95%, 96%, 97%, 98%, 99%, 99,5%, 99,9% oder 99,99% und die Angabe "arm" für einen Gehalt von höchstens 5%, 4%, 3%, 2%, 1 %, 0,5%, 0,1% oder 0,01% auf molarer, Gewichts- oder Volumenbasis stehen kann. Sind mehrere Komponenten angegeben, bezieht sich die Angabe "reich" oder "arm" auf die Summe aller Komponenten. Ist hier beispielsweise von "Sauerstoff" oder "Ethan" die Rede, kann es sich um ein Reingas, aber auch ein an der jeweiligen Komponente reiches Gemisch handeln.

Stoffströme, Gasgemische usw. können im hier verwendeten Sprachgebrauch außerdem "angereichert" oder "abgereichert" an einer oder mehreren Komponenten sein, wobei sich diese Begriffe auf einen Gehalt in einem Ausgangsgemisch beziehen. Sie sind "angereichert", wenn sie zumindest den 1,5-fachen, 2-fachen, 5-fachen, 10-fachen, 100-fachen oder 1.000-fachen Gehalt, "abgereichert", wenn sie höchstens den 0,75-fachen, 0,5-fachen, 0,1-fachen, 0,01-fachen oder 0,001-fachen Gehalt einer oder mehrerer Komponenten, bezogen auf das Ausgangsgemisch, enthalten.

Nachfolgend werden zur Charakterisierung von Drücken und Temperaturen die Begriffe "Druckniveau" und "Temperaturniveau" verwendet, wodurch zum Ausdruck gebracht werden soll, dass Drücke und Temperaturen nicht in Form exakter Druck- bzw. Temperaturwerte vorliegen werden müssen. Ein Druckniveau oder Temperaturniveau kann beispielsweise ± 1%, 5%, 10%, 20% oder 50% um einen Mittelwert liegen. Mehrere Druck- und Temperaturniveaus können disjunkte oder überlappende Bereiche darstellen. Dasselbe Druck- bzw. Temperaturniveau kann beispielsweise auch dann noch vorliegen, wenn sich aufgrund von Leitungsverlusten oder Abkühlung Drücke und Temperaturen reduziert haben. Bei hier in bar angegebenen Druckniveaus handelt es sich um Absolutdrücke.

Bei einer "Rektifikationskolonne" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, eingespeistes Stoffgemisch durch Rektifikation zumindest teilweise aufzutrennen, also aus dem Stoffgemisch jeweils Reinstoffe oder zumindest Stoffgemische mit anderer Zusammensetzung zu erzeugen. Typischerweise sind Rektifikationskolonnen als zylindrische Metallbehälter ausgebildet, die mit Einbauten, beispielsweise Trennböden oder geordneten oder ungeordneten Packungen, ausgerüstet sind. Eine Rektifikationskolonne weist einen Sumpfverdampfer auf. Hierbei handelt es sich um eine Einrichtung mit einem Wärmetauscher, der beheizt wird und dafür eingerichtet ist, eine im Sumpf der Rektifikationskolonne anfallende flüssige Fraktion, auch als Sumpfflüssigkeit bezeichnet, zu erwärmen. Mittels eines Sumpfverdampfers wird kontinuierlich ein Teil des Sumpfprodukts verdampft und gasförmig in den Trennbereich zurückgespeist.

### Vorteile der Erfindung

Wie eingangs erwähnt, sind in einem typischen, mittels ODH gebildeten Prozessgas neben Hauptprodukten wie Olefinen (und typischerweise Carbonsäuren, die insbesondere bei Verwendung bestimmter Katalysatoren parallel gebildet werden) unter anderem nicht umgesetzte Paraffine, Sauerstoff, Kohlenmonoxid und Kohlendioxid enthalten. Das Prozessgas enthält typischerweise auch Wasser sowie ggf. geringere Mengen Inertgase. In einer der ODH nachgeschalteten Trennung müssen daher die Nebenprodukte von dem oder den gewünschten Hauptprodukten bzw. den nicht umgesetzten Paraffinen abgetrennt werden.

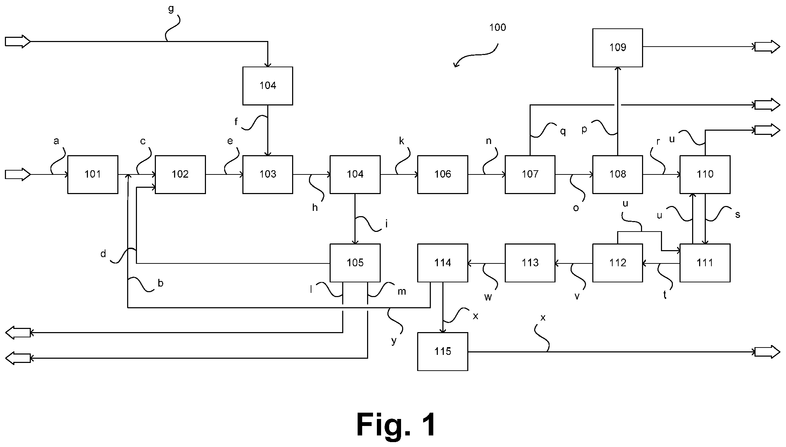

Die Trennung erfolgt, wie auch unter Bezugnahme auf die beigefügte Figur 1 veranschaulicht, typischerweise nach einer Verdichtung des Prozessgases. Dieses wird anschließend typischerweise mittels einer Wasserwäsche zumindest von großen Teilen der ggf. vorhandenen Carbonsäuren und des enthaltenen Wassers befreit. Anschließend erfolgen Waschschritte zur Entfernung von Kohlendioxid.

Das nun noch im Wesentlichen Olefine, die nicht umgesetzten Paraffine, Sauerstoff und Kohlenmonoxid sowie geringere Mengen Inertgase enthaltende Prozessgas wird von Restwasser befreit und vorgekühlt. In einer sich anschließenden Tieftemperaturtrennung erfolgt nun eine stufenweise Kondensation des Prozessgases. Die verbleibenden Gasfraktionen werden jeweils dem nächsten Kondensationsschritt zugeführt. Die Kondensate werden in einer Tieftemperaturrektifikation getrennt, wobei wiederum eine Gasfraktion sowie eine flüssige Fraktion gebildet werden. Die Gasfraktion aus der Tieftemperaturrektifikation wird typischerweise mit der stromab des letzten Kondensationsschritts verbleibenden Gasfraktion zu einer weiteren Gasfraktion, der sogenannten Brenngasfraktion bzw. Tailgasfraktion, vereinigt und typischerweise einer thermischen Nutzung zugeführt. Die flüssige Fraktion der Tieftemperaturrektifikation wird weiteren Trennschritten unterworfen.

Die Brenngasfraktion umfasst typischerweise zumindest den überwiegenden Teil des in dem Prozessgas, das der Tieftemperaturtrennung zugeführt wurde, enthaltenen

Kohlenmonoxids und Sauerstoffs sowie der übrigen, tiefer als die gewünschten Olefine siedenden Komponenten. Die gewünschten Olefine gehen zumindest überwiegend in die flüssige Fraktion aus der Tieftemperaturrektifikation über, gemeinsam mit den höher siedenden nicht umgesetzten Paraffinen.

Aufgrund der Art der kondensativen bzw. destillativen Gewinnung der Gasfraktionen in der Tieftemperaturtrennung reichern sich diese ohne zusätzliche Maßnahmen zunehmend mit Sauerstoff und Kohlenmonoxid an. Ein typisches Prozessgas, das der Tieftemperaturtrennung zugeführt wird, enthält beispielsweise ca. 36 Molprozent Ethan, 37 Molprozent Ethylen, 20 Molprozent Kohlenmonoxid, 4 Molprozent Sauerstoff und 1 Molprozent inerte Komponenten. Unter "inerten Komponenten" werden dabei nachfolgend in der ODH nicht reaktive und unter den verwendeten Bedingungen nicht kondensierbare Komponenten verstanden, darunter die klassischen Inertgase wie Stickstoff und Argon, aber auch beispielsweise Methan, das sich in der ODH ebenfalls inert verhält. Die verbleibende Gasfraktion enthält in diesem Fall bereits nach einem ersten Kondensationsschritt, bei dem das Prozessgas bei ca. 20,4 bar auf -53° C abgekühlt wird, ca. 44 Molprozent Kohlenmonoxid und 8 Molprozent Sauerstoff. In diesem Gasgemisch sind sowohl die Explosionsgrenze als auch die Sauerstoffgrenzkonzentration (siehe sogleich) bereits deutlich überschritten. Allgemein kann bereits ein Sauerstoffanteil von 3000 vppm am Reaktoraustritt dazu führen, dass ein in der Tieftemperaturrektifikation gebildetes Gasgemisch Kohlenmonoxid- und Sauerstoffanteile von 66 Vol.-% bzw. 13 Vol.-% aufweist.

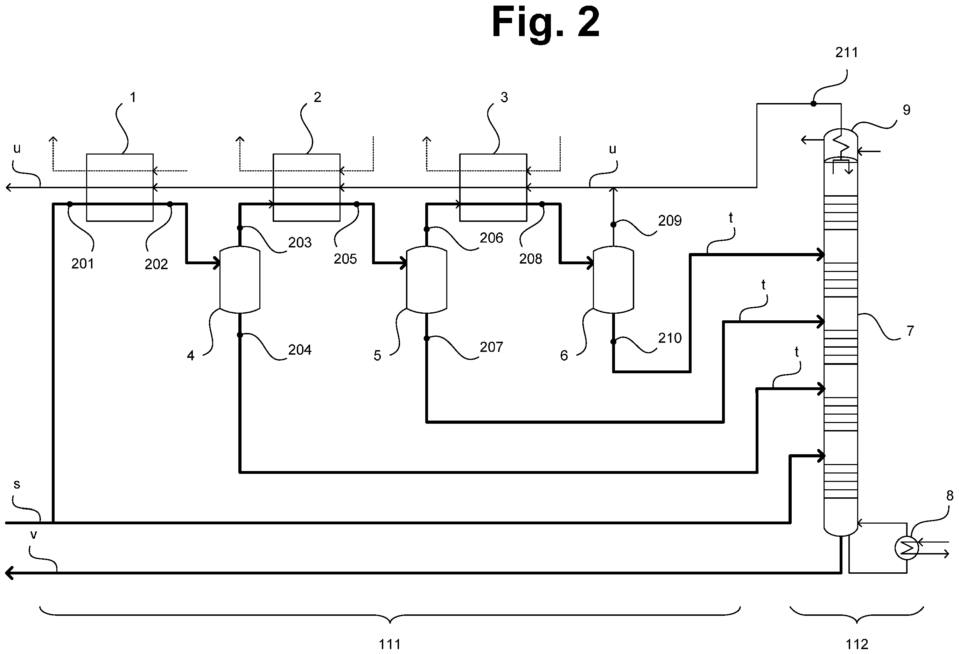

Die (untere) Explosionsgrenze eines Gases gibt den Gehalt in einem Gasgemisch an, ab dem eine Entzündung bzw. Explosion bei zugleich ausreichendem Sauerstoffgehalt möglich ist. Diese liegt für Kohlenmonoxid bei einem Gehalt von deutlich über 10 Molprozent und wird unter anderem durch den Gehalt an Wasserstoff und Wasser in dem jeweils betrachteten Gasgemisch beeinflusst. Unter Standardbedingungen wird sie mit 12,5 Molprozent angegeben. In den erläuterten, in einer typischen Tieftemperaturtrennung stromab einer ODH gebildeten Gasfraktion liegt der Gehalt an Kohlenmonoxid bei mindestens 40 und bis zu 70 Molprozent, wie auch unter Bezugnahme auf die beigefügte Figur 2 erläutert und soeben erwähnt, so dass die Explosionsgrenze deutlich überschritten ist.

Wenn die Explosionsgrenze überschritten ist, kann eine Explosion erfolgen, sofern auch die sogenannte Sauerstoffgrenzkonzentration überschritten ist. Diese gibt den Sauerstoffgehalt an, ab dem eine Explosion eintreten kann. Mit anderen Worten müssen, damit eine Explosion möglich ist, sowohl die Explosionsgrenze als auch die Sauerstoffgrenzkonzentration überschritten sein.

Die Sauerstoffgrenzkonzentration für Kohlenmonoxid ist nun ausgesprochen gering und liegt bei etwa der Hälfte von jener von Methan und etwa bei jener von Ethylen. In Gemischen aus unterschiedlichen brennbaren Gaskomponenten stellen sich typischerweise Zwischenwerte ein. Im vorliegenden Fall, d.h. den in der Tieftemperaturtrennung gebildeten Gasgemischen, liegt die Sauerstoffgrenzkonzentration bei einem Wert von ca. 6 Molprozent, sofern keine weiteren Maßnahmen ergriffen werden. Es kann daher grundsätzlich zu Explosionen und ggf. sogar zu Detonationen kommen.

Die Explosionsgrenzen von Gasen können gemäß standardisierter Verfahren entsprechend einschlägiger Normen wie DIN 51649-1 oder EN 1839 bestimmt werden. Die Explosionsgrenzen zahlreicher brennbarer Gase und Dämpfe in Luft unter Standardbedingungen sind in umfangreichen Tabellenwerken und Datenbanken erfasst. Bei nichtatmosphärischen Bedingungen und ebenso bei Gemischen unterschiedlicher Gase kann eine Extrapolation ausgehend von bekannten Werten erfolgen oder es kann eine Berechnung aufgrund bekannter Vorschriften durchgeführt werden. Für weitere Details sei auf Fachliteratur, beispielsweise Markus, D., und Maas, U., Die Berechnung von Explosionsgrenzen mit detaillierter Reaktionskinetik, Chemie Ingenieur Technik, Bd. 76, Nr. 3, 2004, und die dortigen Zitate verwiesen. Entsprechendes gilt für die Bestimmung von Sauerstoffgrenzkonzentrationen.

Bei einer Explosion handelt es sich um einen unkontrollierten Abbrand eines zündfähigen Gasgemischs mit laminarer Flammenfront. Explosionen und Detonationen unterscheiden sich im Wesentlichen durch die Geschwindigkeit der Ausbreitung. Bei einer Explosion liegt diese unterhalb, bei einer Detonation typischerweise deutlich oberhalb der Schallgeschwindigkeit. Durch Explosionen und Detonationen von Gasgemischen in Behältern kommt es zu einem massiven Druckanstieg, der zum Bersten der Behälter und entsprechenden Folgeschäden führen kann. Für stöchiometrische Luft-/Kohlenwasserstoffgemische kann ein maximaler Druckanstieg bei einer Explosion um den Faktor Zehn errechnet werden. Die Auswirkungen einer Detonation sind deutlich schwerwiegender. Hier kann der Druckanstiegsfaktor 50 und mehr betragen. Eine Explosion kann nach einer bestimmten Anlauflänge und einer Mindestkonzentration an Brennstoff und Sauerstoff in eine Detonation umschlagen.

Die vorliegende Erfindung löst diese Probleme in einem Verfahren zur Herstellung eines oder mehrerer Olefine, bei dem ein Reaktionseinsatz gebildet wird, der ein oder mehrere Paraffine enthält, und bei dem ein Teil des oder der in dem Reaktionseinsatz enthaltenen Paraffine unter Erhalt eines Prozessgases durch oxidative Dehydrierung zu dem oder den Olefinen umgesetzt wird, wobei das Prozessgas zumindest das oder die Olefine, das oder die nicht umgesetzten Paraffine, Sauerstoff und Kohlenmonoxid enthält, und wobei zumindest ein Teil des Prozessgases einer Tieftemperaturtrennung unterworfen wird, in der auf einem Betriebsdruckniveau eine oder mehrere gegenüber dem Prozessgas an Sauerstoff und Kohlenmonoxid angereicherte Gasfraktionen gebildet werden, dadurch, dass in der Tieftemperaturtrennung bei der Bildung und/oder zur Führung der oder zumindest einer der Gasfraktionen ein oder mehrere Behälter und/oder eine oder mehrere Leitungen mit einem Berstdruck verwendet werden, der zumindest dem Zehnfachen des Betriebsdruckniveaus entspricht.

Das Betriebsdruckniveau bezeichnet dabei das im regulären Betrieb in der Tieftemperaturtrennung eingesetzte Druckniveau, das beispielsweise bei 10 bis 30 bar liegen kann. Unter einem "Berstdruck" wird nachfolgend insbesondere ein rechnerischer Berstdruck, der beispielsweise aus Material- und Konstruktionsdaten ermittelt wird, und/oder ein Prüfdruck und/oder ein mechanischer Auslegungsdruck (für explosionsfeste Auslegung) verstanden.

Ein "Reaktionseinsatz" ist im hier verwendeten Sprachgebrauch das gesamte, der ODH unterworfene Gasgemisch. Dieses kann insbesondere auch in Form separater Stoffströme dem oder den verwendeten Reaktoren zugespeist werden. Beispielsweise können ein paraffinhaltiger Stoffstrom und ein sauerstoffhaltiger Stoffstrom zu einem entsprechenden Reaktionseinsatz in dem oder den verwendeten Reaktoren oder stromauf des oder der Reaktoren vereinigt werden.

Im Rahmen der vorliegenden Erfindung ist damit zumindest ein Teil der Behälter und Leitungen, in denen entsprechende Gasfraktionen mit hohem Sauerstoff- und
Kohlendioxidgehalt vorliegen, explosionsfest ausgelegt. Wie erwähnt, erfolgt bei einer Explosion der erwähnten Gasfraktionen typischerweise ein Druckanstieg um maximal den Faktor zehn. Die erfindungsgemäß vorgeschlagenen Maßnahmen stellen sicher, dass trotz der Zusammensetzung entsprechender Gasfraktionen und auch bei deren Explosion keine Beschädigungen der drucktragenden Bauteile einer entsprechenden Anlage mehr auftreten können und/oder keine Freisetzung von entsprechenden Gasfraktionen oder deren Verbrennungsprodukten an die Umgebung erfolgt. Auf diese Weise kann auf verfahrenstechnische Maßnahmen wie die Zudosierung von Inertisierungsmitteln oder die Ausgestaltung der Trennung sowie die Verwendung von Explosionssperren zumindest teilweise verzichtet werden.

Wie bereits zuvor erläutert, wird hier unter einer entsprechenden "Gasfraktion" insbesondere ein Gasgemisch verstanden, das gegenüber dem Prozessgas an Sauerstoff und Kohlenmonoxid angereichert ist, und das in einer Tieftemperaturtrennung gebildet wird. Bei einem Abstrom eines stromauf der Tieftemperaturtrennung, beispielsweise in einer Wasserwäsche oder einer Kohlendioxidentfernung, gebildeten Gasgemisch handelt es sich damit nicht um eine Gasfraktion in diesem Sinne, da diese nicht in der Tieftemperaturtrennung gebildet wird. Entsprechende Gasfraktionen fallen insbesondere in den erwähnten Kondensationsschritten und der nachgeschalteten Tieftemperaturrektifikation der gewonnenen Kondensate an. Auch bei der erläuterten Brenngasfraktion handelt es sich um eine in der Tieftemperaturtrennung gebildete Gasfraktion.

Aus dem Stand der Technik ist zur Vermeidung von Explosionen beispielsweise bekannt, die Anreicherung von Sauerstoff und/oder brennbarer Anteile in entsprechenden Gasfraktionen zu vermeiden und dabei den Gehalt an entsprechenden Komponenten kontinuierlich zu überwachen. Gegebenenfalls wird eine entsprechende Gasfraktion verworfen und/oder die vorgeschaltete Trennung wird weniger scharf ausgeführt, um die beschriebene Aufkonzentration zu vermeiden. Im Ergebnis führt dies zu einer verringerten Gesamtausbeute, da Produkt mit verworfen wird.

Im Rahmen der vorliegenden Erfindung wird durch die explosionsfeste Auslegung dagegen die Gesamtausbeute erhöht. Im Rahmen der vorliegenden Erfindung müssen nicht notwendigerweise sämtliche Behälter und/oder Leitungen, in denen explosionsfähige Gasfraktionen vorliegen, explosionsfest ausgeführt werden. Es kann auch eine Beschränkung auf solche Behälter und/oder Leitungen erfolgen, die einem höheren Explosionsrisiko ausgesetzt sind als andere.

Bei langen Rohrleitungen und/oder Behältern (d.h. dann, wenn eine freie Wegstrecke, die zur Ausbreitung einer Explosion zur Verfügung steht, und die sich aus der Rohrleitungs- und ggf. der Behälterlänge zusammensetzt, mindestens dem Fünfzigfachen des Innendurchmessers entspricht) kann die Explosion eines Kohlenwasserstoff/Sauerstoff-Gemisches in eine Detonation umschlagen, weil hier eine ausreichende Anlauflänge vorliegt. Dies trifft insbesondere dann zu, wenn entsprechende stöchiometrische Verhältnisse (Lambdawert eins, ideale Mischung) vorliegen. Der Umschlag einer Explosion in eine Detonation wird nach Stand dem Technik in der Petrochemie in unter Druck stehenden Systemen beispielsweise durch das Vorsehen von Einbauten, die Einbringung von Schüttungen, den Einbau von Explosionsschutzfiltern, die Ausführung von Rohrleitungen als Bündel, den Einbau von Löschsystemen (Staub) und die Verwendung von Flüssigkeitsschleiern verhindert. All diese Lösungen haben gemein, dass die Explosion durch eine hohe Masse bei kleinen Spaltweiten und großer Oberfläche zum Erliegen gebracht wird. Die sich ausbreitenden Flamme wird abgekühlt und erlischt im Idealfall schließlich. Allen diesen Maßnahmen sind kleine Spaltmaße bei großer Oberfläche gemein. Alle diese Maßnahmen führen zu erhöhten Gestehungskosten, Betriebs- und Wartungskosten.

Gemäß einer besonders bevorzugten Ausgestaltung der vorliegenden Erfindung wird eine Detonation vermieden, indem entsprechende Leitungen und/oder Behälter ausreichend kurz gehalten werden. Insbesondere wurde in diesem Zusammenhang erkannt, dass die in der genannten Tieftemperaturtrennung eingesetzten Wärmetauscher, mit denen entsprechende Leitungen verbunden sind, als Explosionssperren ausgebildet werden können oder inhärent effektive Explosionssperren darstellen. Vorteilhafterweise ist daher vorgesehen, dass zumindest einer der Behälter über die oder zumindest eine der Leitungen mit einem oder mehreren Wärmetauschern verbunden ist, wobei eine Gesamtlänge der oder der zumindest einen Leitung zwischen dem oder dem zumindest einen Behälter und dem oder den Wärmetauschern höchstens das Fünfzigfache ihres Innendurchmessers beträgt. Die genannten Leitungen und/oder Behälter sind vorteilhafterweise weiterhin explosionsfest ausgelegt, aufgrund der reduzierten Leitungslänge braucht vorteilhafterweise aber keine Detonationsfestigkeit hergestellt werden.

Es ist also von besonderem Vorteil, wenn der oder die Wärmetauscher, mit dem oder denen die oder die zumindest eine Leitung verbunden ist, deren Gesamtlänge zwischen dem oder dem zumindest einen Behälter und dem oder den Wärmetauschern höchstens dem Fünfzigfachen ihres Innendurchmessers entspricht, explosionsausbreitungshemmend ausgebildet sind. Ein entsprechender Wärmetauscher kann beispielsweise dadurch explosionsausbreitungshemmend ausgebildet sein, dass ein effektives Spaltmaß derart gering gehalten wird, dass, wie bei herkömmlichen Einbauten, Schüttungen, Explosionsschutzfiltern usw. eine Explosion durch eine hohe Masse bei kleinen Spaltweiten und großer Oberfläche zum Erliegen gebracht wird. Die sich ausbreitenden Flamme wird auch in einem entsprechenden Wärmetauscher abgekühlt und erlischt schließlich. Auch die explosionsausbreitungshemmende Ausbildung eines Wärmetauschers beruht daher auf der Verwendung kleiner Spaltmaße bei großer Oberfläche.

Um eine geeignete Wahl eines Spaltmaßes zu treffen, bedient sich der Fachmann beispielsweise der bekannten und für eine Vielzahl von Gasen oder Gasgemischen publizierten Normspaltweiten. Die Normspaltweite ist die größte Weite eines 25 mm langen Spaltes, die unter festgelegten Prüfbedingungen sicher verhindert, dass ein in einem Gerät gezündetes Gasgemisch ein außerhalb des Gerätes befindliches Gasgemisch durch den Spalt hindurch zündet. Entsprechende Normspaltweiten können ohne weiteres auf die bei Wärmetauschern verwendeten Spaltmaße übertragen bzw. umgerechnet werden. Vorteilhafterweise ist ein entsprechender Wärmetauscher explosionsfest ausgeführt, d.h. er weist einen Berstdruck auf, der zumindest dem Zehnfachen des Betriebsdruckniveaus entspricht. Auf diese Weise kann eine Explosion eintreten, ohne dass diese in eine Detonation umschlagen und zu Beschädigungen führen kann.

Es ist weiter von besonderem Vorteil, wenn Gase oder Zweiphasengemische führende Leitungen, die mit Behältern verbunden sind, in denen Kondensate abgeschieden werden, abgetaucht münden, also unter dem gewünschten Flüssigkeitsspiegel enden. Hierbei perlt das durchströmende Gas durch eine Flüssigkeit, wodurch eine Flüssigkeitsexplosionssperre in den Behälter integriert wird.

Es ist weiter von besonderem Vorteil, Kolonneneinbauten als gerichtete Packungen oder Schüttungen auszuführen, wodurch eine Explosionssperre in die Kolonnnenfunktion integriert wird.

Es ist weiter von besonderem Vorteil, wenn das in der Kolonne enthaltene Gesamtgasvolumen durch mindestens einen Rektifikationsboden in mehrere durch Flüssigkeit und ggf. feste Einbauten (z.B. Schachtwände) voneinander getrennte Gasvolumina aufgeteilt wird, wodurch eine Flüssigkeitsexplosionssperre in die Kolonnenfunktion integriert wird. Dies kann beispielsweise durch einen oder zumindest den einen ein- oder mehrflutigen, in Kreuzstrom betriebenen Rektifikationsboden erfolgen.

Die vorliegende Erfindung kann gleichwohl auch dann vorteilhafterweise zum Einsatz kommen, wenn der oder zumindest einer der Behälter über die oder zumindest eine der Leitungen mit einem oder mehreren Wärmetauschern verbunden ist, aber eine Gesamtlänge der oder der zumindest einen Leitung zwischen dem oder dem zumindest einen Behälter und dem oder den Wärmetauschern mehr als das Fünfzigfache ihres Innendurchmessers beträgt. Eine entsprechende Leitung kann, insbesondere in explosionskritischen Umgebungen, in diesem Fall mit einer Explosionssperre versehen werden, um eine Ausbreitung der Explosion und ein Umschlagen in eine Detonation zu verhindern. Es ist auch möglich, entsprechende Leitungen detonationsfest auszuführen. Eine entsprechende Leitung weist dann einen Berstdruck auf, der zumindest dem Fünfzigfachen des Betriebsdruckniveaus entspricht

Im Rahmen der vorliegenden Erfindung kann insbesondere eine Tieftemperaturtrennung eingesetzt werden, wie sie bereits zuvor erläutert wurde. Vorteilhafterweise umfasst also die Tieftemperaturtrennung, das Prozessgas mehrfach und stufenweise abzukühlen, wobei nach jeder Abkühlung unter Verbleib einer der Gasfraktionen ein Kondensat aus dem Prozessgas abgeschieden und zumindest ein Teil der Gasfraktionen einer weiteren Abkühlung zugeführt wird. Wie erwähnt, kann gerade in diesen Gasfraktionen eine entsprechende Anreicherung auftreten, die entsprechende Gasfraktionen enthaltenden bzw. führenden Behälter und Leitungen werden daher vorteilhafterweise wie erwähnt ausgeführt. Mit anderen Worten werden zum Abscheiden zumindest eines der Kondensate der oder zumindest einer der Behälter und zum Zuführen zumindest einer der Gasfraktionen zu der weiteren Abkühlung die oder eine der Leitungen verwendet.

Eine Anlage zur Herstellung eines oder mehrerer Olefine, die dafür eingerichtet ist, einen Reaktionseinsatz zu bilden, der ein oder mehrere Paraffine enthält, mit einer Reaktionseinheit, die dafür eingerichtet ist, einen Teil des oder der in dem Reaktionseinsatz enthaltenen Paraffine unter Erhalt eines Prozessgases durch oxidative Dehydrierung zu dem oder den Olefinen umzusetzen, wobei das Prozessgas zumindest das oder die Olefine, das oder die nicht umgesetzten Paraffine, Sauerstoff und Kohlenmonoxid enthält, und mit einer Tieftemperaturtrennung, die dafür eingerichtet ist, auf einem Betriebsdruckniveau eine oder mehrere gegenüber dem Prozessgas an Sauerstoff und Kohlenmonoxid angereicherte Gasfraktionen zu bilden, ist ebenfalls Gegenstand der Erfindung.

Eine derartige Anlage zeichnet sich dadurch aus, dass in der Tieftemperaturtrennung zur Bildung und/oder zur Führung der oder zumindest einer der Gasfraktionen ein oder mehrere Behälter und/oder eine oder mehrere Leitungen mit einem Berstdruck vorgesehen sind, der zumindest dem Zehnfachen des Betriebsdrucksniveaus entspricht.

Zu Merkmalen und Vorteilen einer entsprechenden Anlage sei auf die obigen Erläuterungen zu den Merkmalen und Vorteilen des Verfahrens verwiesen. Insbesondere ist eine derartige Anlage zur Durchführung eines Verfahrens gemäß den oben erläuterten spezifischen Ausgestaltungen eingerichtet und weist hierzu geeignete Mittel auf. Auch diesbezüglich sei auf die obigen Ausführungen verwiesen.

Die Erfindung wird nachfolgend unter Bezugnahme auf die beigefügten Zeichnungen näher erläutert, die unter anderem bevorzugte Ausführungsformen der vorliegenden Erfindung veranschaulichen.

Kurze Beschreibung der Zeichnungen
Figur 1 veranschaulicht eine Anlage zur Herstellung von Olefinen gemäß einer Ausführungsform der Erfindung.
Figur 2 veranschaulicht eine Rektifikationseinheit zum Einsatz in einer Anlage zur Herstellung von Olefinen gemäß einer Ausführungsform der Erfindung.

### Ausführliche Beschreibung der Zeichnungen

In den nachfolgenden Figuren sind einander funktionell oder baulich entsprechende Elemente mit identischen Bezugszeichen angegeben und werden der Übersichtlichkeit halber nicht wiederholt erläutert. Werden nachfolgend Anlagenteile beschrieben, gelten die Erläuterungen zu diesen sinngemäß auch für die mittels dieser Anlagenteile implementierten Verfahrensschritte und umgekehrt.

In Figur 1 ist eine Anlage zur Herstellung von Olefinen gemäß einer Ausführungsform der Erfindung in Form eines stark vereinfachten Anlagendiagramms veranschaulicht und insgesamt mit 100 bezeichnet. Wenngleich nachfolgend eine Anlage 100 zur ODH von Ethan (ODH-E) beschrieben wird, eignet sich, wie erwähnt, die vorliegende Erfindung auch zum Einsatz bei der ODH höherer Kohlenwasserstoffe. In diesem Fall gelten die nachfolgenden Erläuterungen entsprechend.

In der Anlage 100 wird einer Rektifikationseinheit 101 mit beispielsweise einer oder mehreren Rektifikationskolonnen ein Trenneinsatz in Form eines Stoffstroms a zugeführt und einer Rektifikation unterworfen. Der Trenneinsatz enthält im dargestellten Beispiel zumindest Ethan und höhere Kohlenwasserstoffe, insbesondere entsprechende höhere Paraffine. Der Rektifikationseinheit 101 können auch ein oder mehrere weitere Trenneinsätze zugeführt werden, beispielsweise der hier gezeigte und unten näher erläuterte Stoffstrom b.

In der Rektifikationseinheit 101 wird der Trenneinsatz alleine oder zusammen mit dem oder den weiteren Trenneinsätzen unter Erhalt eines Gasgemischs, das Ethan enthält, aber arm an höheren Kohlenwasserstoffen ist, einer Rektifikation unterworfen. Das Gasgemisch wird in Form eines Stoffstroms c abgezogen und einer Vorwärmeinheit 102 zugeführt. In der Vorwärmeinheit 102 wird das Gasgemisch vorgewärmt, wobei der Vorwärmeinheit 102 im dargestellten Beispiel auch ein Wasser- oder Dampfstrom d zugeführt wird. Auch weitere Stoffströme können zugeführt werden, wie hier in Form eines Stoffstroms b veranschaulicht. In der Rektifikationseinheit 101 wird ferner ein Komponentengemisch erhalten, das überwiegend oder ausschließlich die höheren Kohlenwasserstoffe enthält. Dieses ist nicht explizit gezeigt.

Ein aus der Vorwärmeinheit 102 abströmender Stoffstrom e wird zur Bildung eines Reaktionseinsatzes einer Reaktionseinheit 103 zugeführt. Der Reaktionseinsatz enthält aufgrund seiner Bildung unter Verwendung des Trennprodukts aus der Rektifikationseinheit 101 Ethan, ist aber arm an höheren Kohlenwasserstoffen. Der Reaktionseinsatz kann ferner ein oder mehrere Verdünnungsmittel wie Wasser oder Inertgase und weitere Komponenten enthalten. Diese können der Reaktionseinheit 103 auch in Form weiterer, nicht dargestellter Stoffströme zugeführt werden.

Der Reaktionseinheit 103 wird im dargestellten Beispiel ein sauerstoffhaltiger Stoffstrom f zugeführt. Dieser kann unter Verwendung einer Luftzerlegungsanlage 104 bereitgestellt werden. Der Luftzerlegungsanlage 104 wird hierzu ein Luftstrom g zugeführt. Bei dem sauerstoffhaltigen Stoffstrom f kann es sich um im Wesentlichen reinen Sauerstoff handeln, je nach Betrieb der Luftzerlegungsanlage 104 können jedoch auch Anteile an Stickstoff und an Edelgasen enthalten sein. Auf diese Weise ist es ebenfalls möglich, Verdünnungsmittel zuzuführen.

Aus der Reaktionseinheit 103 strömt ein Prozessgas in Form eines Prozessgasstroms h ab, in dem Ethylen enthalten ist, das in der Reaktionseinheit 103 durch ODH eines Teils des Ethans in dem Reaktionseinsatz gebildet wurden. Ferner enthält das Produktgemisch Essigsäure, die ebenfalls bei der ODH in der Reaktionseinheit 103 aus Ethan gebildet wurde, Wasser, Kohlenmonoxid, Kohlendioxid, nicht umgesetzten Sauerstoff, sowie das oder die Verdünnungsmittel und weitere Verbindungen, falls zugegeben oder zuvor in der Reaktionseinheit 103 gebildet.

Es versteht sich, dass Reaktionseinheit 103 einen, aber auch mehrere, beispielsweise parallel betriebene, Reaktoren aufweisen kann. In letzterem Fall werden diesen Reaktoren jeweils entsprechende Reaktionseinsätze, die gleich oder unterschiedlich zusammengesetzt sein können, sowie entsprechende sauerstoffhaltige Stoffströme f zugeführt werden, und es werden jeweils entsprechende Prozessgasströme h gebildet. Letztere können beispielsweise vereinigt und gemeinsam als Prozessgas den nachfolgend erläuterten Einheiten zugeführt werden.

Das Prozessgas wird in eine Quencheinheit 104 überführt, in der es, beispielsweise in einer Waschsäule, mit Waschwasser oder einer geeigneten wässrigen Lösung in Kontakt gebracht werden kann. In der Quencheinheit 104 wird das Prozessgas insbesondere abgekühlt und die in der Reaktionseinheit 103 gebildete Essigsäure wird aus dem Prozessgas ausgewaschen. Mit Essigsäure beladenes Prozesswasser strömt in Form eines Stoffstroms i aus der Quencheinheit 104 ab, das zumindest weitgehend von Essigsäure befreite Prozessgas in Form eines Stoffstroms k.

In einer optionalen Essigsäurerückgewinnungseinheit 105 wird aus dem mit Essigsäure beladenen Prozesswasser Essigsäure als Eisessig abgetrennt, welcher als Stoffstrom I aus der Anlage 100 ausgeführt wird. In der Essigsäurerückgewinnungseinheit 105 ebenfalls rückgewonnenes reines Prozesswasser kann in Form des bereits erläuterten Stoffstroms d der Vorwärmeinheit 102 zugeführt werden. Das dem Reaktor zugeführte Prozesswasser kann auch teilweise oder ganz durch extern zugeführtes Frischwasser bereitgestellt werden. Nicht mehr brauchbares oder benötigtes Wasser kann in Form eines Abwasserstroms m aus der Anlage 100 ausgeführt und einer Abwasseraufbereitung zugeführt werden.

Das in Form des Stoffstroms k vorliegende, zumindest weitgehend von Essigsäure befreite Prozessgas wird in einer Verdichtungseinheit 106 auf ein geeignetes Druckniveau, beispielsweise 15 bis 25 bar, verdichtet und in Form eines verdichteten Stoffstroms n einer Aminwäscheeinheit 107 zugeführt. In dieser werden insbesondere Teile des in dem Prozessgas enthaltenen Kohlendioxids ausgewaschen. Nach Regenerierung des Amins kann das ausgewaschene Kohlendioxid in Form eines Stoffstroms q aus der Anlage ausgeführt werden. Das derart teilweise von Kohlendioxid befreite Prozessgas wird in Form eines Stoffstroms o in eine Laugenwäscheeinheit 108 überführt und dort weiter von Kohlendioxid gereinigt. In der Laugenwäscheeinheit 108 fällt Ablauge an, die in Form eines Stoffstroms p in eine Ablaugebehandlungseinheit 109 überführt und schließlich aus der Anlage 100 ausgeführt werden kann.

Das in der Laugenwäscheeinheit 108 weiter gereinigte Prozessgas wird in Form eines Stoffstroms r in eine Vorkühl- und Trockeneinheit 110 überführt, wo es insbesondere von Restwasser befreit werden kann. Das getrocknete Prozessgas wird in Form eines Stoffstroms s in eine Tieftemperatureinheit 111 überführt und anschließend in weiter abgekühlter Form in Form eines oder mehrerer Stoffströme t in eine Demethanisierungseinheit 112. In der Tieftemperatureinheit 111 und der Demethanisierungseinheit 112 werden tiefer als Ethylen siedende Komponenten, darunter insbesondere Kohlenmonoxid und Sauerstoff, aus dem Prozessgas abgetrennt, wobei der Rest in kondensierter Form verbleibt. Sind in dem Prozessgas höhere Kohlenwasserstoffe enthalten, die bei der ODH in der Reaktionseinheit 103 als Nebenprodukt gebildet wurden, gehen diese ebenfalls in das Kondensat über.

Die abgetrennten, tiefer als Ethylen siedenden Komponenten werden in Form eines oder mehrerer Stoffströme u durch die Tieftemperatureinheit 111 und die Vorkühl- und Trockeneinheit 110 zurückgeführt, dort ggf. mit weiteren entsprechender Stoffströme vereinigt, zu Kühlzwecken genutzt, und aus der Anlage 100 ausgeführt. Die Kohlenwasserstoffe mit zwei und ggf. mehr Kohlenstoffatomen werden bei Bedarf in Form eines Stoffstroms v einer Hydriereinheit 113 zugeführt, in der insbesondere Acetylen, das ebenfalls bei der ODH in der in der Reaktionseinheit 103 als Nebenprodukt gebildet wurde, hydriert werden kann. Nach der Hydrierung wird der nun mit w bezeichnete Stoffstrom in eine Ethylenabtrenneinheit 114 überführt. Details zu einer verwendbaren Tieftemperatureinheit 111 und zu einer verwendbaren Demethanisierungseinheit 112 sind in Figur 2 näher veranschaulicht.

In der Ethylenabtrenneinheit 114 wird Ethylen zumindest weitgehend von anderen Komponenten abgetrennt und kann in Form eines Stoffstroms x nach Nutzung in einer Ethylenkälteeinheit 115 gasförmig aus der Anlage 100 ausgeführt werden. Die übrigen Komponenten, überwiegend Ethan und ggf. höhere Kohlenwasserstoffe, werden in Form eines Stoffstroms y abgezogen. Sind in diesem höhere Kohlenwasserstoffe enthalten, werden diese vorteilhafterweise in Form des bereits erwähnten Stoffstroms b in die Reaktionseinheit zurückgeführt. Es kann eine optionale Aufbereitung dieses Stoffstroms b erfolgen.

Figur 2 zeigt eine Tieftemperatureinheit und eine Demethanisierungseinheit zum Einsatz in einer Anlage zur Herstellung von Olefinen gemäß einer Ausführungsform der Erfindung. Die Tieftemperatureinheit und die Demethanisierungseinheit können beispielsweise in einer Anlage 100, wie sie in Figur 1 gezeigt ist, zum Einsatz kommen, und sind daher wie dort mit 111 und 112 zusammengefasst. Die bereits in Figur 1 dargestellten Stoffströme s, t, u und v sind auch hier gezeigt. Die Darstellung der jeweiligen Elemente ist nicht positions- und nicht maßstabsgerecht.

Das teilkondensierte Prozessgas wird in Form des Stoffstroms s der Tieftemperatureinheit 111 zugeführt. Der gasförmige Teil des Prozessgases wird nacheinander durch Wärmetauscher 1 bis 3 geführt und dort auf immer niedrigere Temperaturniveaus gekühlt. Die Wärmetauscher 1 bis 3 werden dazu mit gestrichelt gezeigten Ethylenströmen gekühlt (Hochdruckethylen in Wärmetauscher 1, Mitteldruckethylen in Wärmetauscher 2, Niederdruckethylen in Wärmetauscher 3). Zur Kühlung wird zusätzlich der Stoffstrom u eingesetzt, der im dargestellten Beispiel die tiefer als Ethan, bzw. allgemeiner, die tiefer als die Paraffine mit N Kohlenstoffatomen siedenden Komponenten des Prozessgases enthält, die in der Tieftemperatureinheit 111 und der Demethanisierungseinheit 112 abgetrennt werden.

Stromab der Wärmetauscher 1 bis 3 wird das Prozessgas bzw. ein durch die Abkühlung in den Wärmetauschern 1 bis 3 jeweils gebildetes Zweiphasengemisch jeweils in Abscheider 4 bis 6 überführt, wo sich jeweils ein Kondensat aus dem Prozessgas abscheidet. Die Kondensate werden in ihrer stofflichen Zusammensetzung entsprechender Höhe in eine Rektifikationskolonne 7 der Demethanisierungseinheit 112, den sogenannten Demethanizer, eingespeist. Ferner wird der bereits in der Vorkühlung kondensierte Anteil des Prozessstroms s direkt in die Rektifikationskolonne 7 eingespeist. Es kann auch eine Trennung von flüssigen und gasförmigen Anteilen des Prozessstroms s vor der Einspeisung erfolgen.

Ein Sumpfverdampfer 8 der Rektifikationskolonne 7 wird beispielsweise unter Verwendung von Propan beheizt, ein Kopfkondensator 9 beispielsweise unter Verwendung von Niederdruckethylen gekühlt. Die Rektifikationskolonne 7 wird derart betrieben, dass sich an ihrem Kopf überwiegend die tiefer als Ethylen siedenden Komponenten und in ihrem Sumpf die schwereren Verbindungen anreichern. Auf diese Weise kann vom Kopf der Rektifikationskolonne 7 ein Teil des Stoffstroms u und aus dem Sumpf der Rektifikationskolonne 7 der Stoffstrom v abgezogen werden. Ein in dem Abscheider 6 gasförmig verbliebener Anteil des Prozessgases kann ebenfalls bei der Bildung des Stoffstroms u verwendet werden.

In den nachfolgenden Tabellen 1 und 2 sind typische Parameter einer Auswahl der in Figur 2 gezeigten Stoffströme an Positionen 201 bis 211 der Tieftemperatureinheit 111 und der Demethanisierungseinheit 112 veranschaulicht, wie sie sich ohne Zuspeisung weiterer Gase wie zuvor erläutert ergeben.

Das Prozessgas, das in Form des Stoffstroms s eingespeist wird, weist im dargestellten Beispiel an Position 201 neben den in Tabelle 2 angegebenen Gehalten an Kohlenmonoxid und Sauerstoff 36 Molprozent Ethan, 37 Molprozent Ethylen und 1 Molprozent Argon auf. In dem vom Kopf der Rektifikationskolonne 7 abgezogenen Gasgemisch sind an Position 211 neben den in Tabelle 2 angegebenen Gehalten an Kohlenmonoxid und Sauerstoff außerdem 10 Molprozent Ethylen und 4 Molprozent Argon enthalten. Die genannten Werte sind typische Beispielwerte.

**Tabelle 1**

| **Position** | **Druck** | **Temperatur** |
|---|---|---|
| 201 | 20,4 bar | -31°C |
| 202 | 20,2 bar | -53°C |
| 205 | 19,9 bar | -76 °C |
| 208 | 19,7 bar | -97 °C |
| 211 | 18,8 bar | -97 °C |

**Tabelle 2**

| **Position** | **Kohlenmonoxid** | **Sauerstoff** | **Durchsatz** |
|---|---|---|---|
| 201 | 20 mol-% | 4 mol-% | 3 t/h |
| 203 | 44 mol-% | 8 mol-% | 1,2 t/h |
| 204 | 3,8 mol-% | 0,8 mol-% | 1,2 t/h |
| 206 | 62 mol-% | 11 mol-% | 0,8 t/h |
| 207 | 5 mol-% | 1,3 mol-% | 0,4 t/h |
| 209 | 72 mol-% | 13 mol-% | 0,7 t/h |
| 210 | 8 mol-% | 2 mol-% | 0,1 t/h |
| 211 | 64 mol-% | 13 mol-% | 2,6 t/h |

Wie aus Tabelle 2 ersichtlich, erreicht der Sauerstoffgehalt ohne Zuspeisung weiterer Gase wie zuvor erläutert bereits an Position 203 einen Wert, der mit dem enthaltenen Kohlenmonoxid ein explosionsfähiges Gemisch ergibt.

Die vorliegende Erfindung sieht dass in der Tieftemperaturtrennung bei der Bildung und/oder zur Führung der oder zumindest einer der Gasfraktionen ein oder mehrere Behälter und/oder eine oder mehrere Leitungen mit einem Berstdruck verwendet werden, der zumindest dem Zehnfachen des Betriebsdruckniveaus entspricht. Bei solchen Leitungen kann es sich beispielsweise um die Behälter 4 bis 6 und die Leitungen an den Positionen 201 bis 211 handeln, die, wie erwähnt, explosive Gasgemische führen können. Die Rektifikationskolonne 7 wird vorteilhafterweise ebenfalls explosionsfest ausgebildet.

## Patentansprüche

1. Verfahren zur Herstellung eines oder mehrerer Olefine, bei dem ein Reaktionseinsatz gebildet wird, der ein oder mehrere Paraffine enthält, und bei dem ein Teil des oder der in dem Reaktionseinsatz enthaltenen Paraffine unter Erhalt eines Prozessgases durch oxidative Dehydrierung zu dem oder den Olefinen umgesetzt wird, wobei das Prozessgas zumindest das oder die Olefine, das oder die nicht umgesetzten Paraffine, Sauerstoff und Kohlenmonoxid enthält, und wobei zumindest ein Teil des Prozessgases einer Tieftemperaturtrennung unterworfen wird, in der auf einem Betriebsdruckniveau eine oder mehrere gegenüber dem Prozessgas an Sauerstoff und Kohlenmonoxid angereicherte Gasfraktionen gebildet werden, **dadurch gekennzeichnet, dass** in der Tieftemperaturtrennung bei der Bildung und/oder zur Führung der oder zumindest einer der Gasfraktionen ein oder mehrere Behälter und/oder eine oder mehrere Leitungen mit einem Berstdruck verwendet werden, der zumindest dem Zehnfachen des Betriebsdruckniveaus entspricht.

2. Verfahren nach Anspruch 1, bei dem der oder zumindest einer der Behälter über die oder zumindest eine der Leitungen mit einem oder mehreren Wärmetauschern verbunden ist, wobei eine Gesamtlänge der oder der zumindest einen Leitung zwischen dem oder dem zumindest einen Behälter und dem oder den Wärmetauschern höchstens das Fünfzigfache ihres Innendurchmessers beträgt.

3. Verfahren nach Anspruch 2, bei dem der oder die Wärmetauscher, mit dem oder denen die oder die zumindest eine Leitung verbunden ist, deren Gesamtlänge zwischen dem oder dem zumindest einen Behälter und dem oder den Wärmetauschern höchstens dem Fünfzigfachen ihres Innendurchmessers entspricht, explosionsausbreitungshemmend ausgebildet sind.

4. Verfahren nach Anspruch 3, bei dem die explosionsausbreitungshemmende Ausbildung des oder der Wärmetauscher die Verwendung eines auf die Gasfraktion(en) abgestimmten Höchstspaltmaßes umfasst.

5. Verfahren nach Anspruch 3 oder 4, bei dem der oder die explosionsausbreitungshemmend ausgebildeten Wärmetauscher einen Berstdruck aufweisen, der zumindest dem Zehnfachen des Betriebsdruckniveaus entspricht.

6. Verfahren nach einem der vorstehenden Ansprüche, bei dem der Berstdruck ein rechnerischer Berstdruck und/oder ein Prüfdruck und/oder ein mechanischer Auslegungsdruck ist.

7. Verfahren nach Anspruch 1, bei dem der oder zumindest einer der Behälter über die oder zumindest eine der Leitungen mit einem oder mehreren Wärmetauschern verbunden ist, wobei eine Gesamtlänge der oder der zumindest einen Leitung zwischen dem oder dem zumindest einen Behälter und dem oder den Wärmetauschern mehr als das Fünfzigfache ihres Innendurchmessers beträgt.

8. Verfahren nach Anspruch 7, bei dem die oder die zumindest eine Leitung, deren Gesamtlänge zwischen dem oder dem zumindest einen Behälter und dem oder den Wärmetauschern mehr als das Fünfzigfache des Innendurchmessers beträgt, einen Berstdruck aufweist, der zumindest dem Fünfzigfachen des Betriebsdruckniveaus entspricht.

9. Verfahren nach Anspruch 7 oder 8, bei dem die oder die zumindest eine Leitung, deren Gesamtlänge zwischen dem oder dem zumindest einen Behälter und dem oder den Wärmetauschern mehr als das Fünfzigfache des Innendurchmessers beträgt, eine Explosionssperre aufweist.

10. Verfahren nach einem der vorstehenden Ansprüche, bei dem durch die oder zumindest eine der Leitungen ein zumindest teilweise gasförmiges Fluid geführt und in den oder zumindest einen der Behälter eingespeist wird, wobei in dem oder dem zumindest einen Behälter eine Flüssigkeit aus dem Fluid abgeschieden wird und die oder die zumindest eine Leitung unterhalb eines Flüssigkeitsspiegels der Flüssigkeit in den oder den zumindest einen Behälter mündet.

11. Verfahren nach einem der vorstehenden Ansprüche, bei dem die Tieftemperaturtrennung die Verwendung einer Rektifikationskolonne umfasst, die wenigstens eine gerichtete Packung und/oder Schüttung aufweist.

12. Verfahren nach einem der vorstehenden Ansprüche, bei dem die Tieftemperaturtrennung die Verwendung einer Rektifikationskolonne umfasst, die wenigstens einen Rektifikationsboden aufweist, der/die das in der Kolonne enthaltene Gesamtgasvolumen in mehrere durch Flüssigkeit und ggf. feste Einbauten voneinander getrennte Gasvolumina aufteilt.

13. Verfahren nach einem der vorstehenden Ansprüche, bei dem die Tieftemperaturtrennung umfasst, das Prozessgas mehrfach und stufenweise abzukühlen, wobei nach jeder Abkühlung unter Verbleib einer der Gasfraktionen ein Kondensat aus dem Prozessgas abgeschieden und zumindest ein Teil der Gasfraktionen einer weiteren Abkühlung zugeführt wird.

14. Verfahren nach Anspruch 13, bei dem zum Abscheiden zumindest eines der Kondensate der oder zumindest einer der Behälter und/oder zum Zuführen zumindest einer der der weiteren Abkühlung zu der weiteren Abkühlung die oder eine der Leitungen verwendet werden.

15. Anlage (100) zur Herstellung eines oder mehrerer Olefine, die dafür eingerichtet ist, einen Reaktionseinsatz zu bilden, der ein oder mehrere Paraffine enthält, mit einer Reaktionseinheit (103), die dafür eingerichtet ist, einen Teil des oder der in dem Reaktionseinsatz enthaltenen Paraffine unter Erhalt eines Prozessgases durch oxidative Dehydrierung zu dem oder den Olefinen umzusetzen, wobei das Prozessgas zumindest das oder die Olefine, das oder die nicht umgesetzten Paraffine, Sauerstoff und Kohlenmonoxid enthält, und mit einer Tieftemperaturtrennung, die dafür eingerichtet ist, auf einem Betriebsdruckniveau eine oder mehrere gegenüber dem Prozessgas an Sauerstoff und Kohlenmonoxid angereicherte Gasfraktionen zu bilden, **dadurch gekennzeichnet, dass** in der Tieftemperaturtrennung zur Bildung und/oder zur Führung der oder zumindest einer der Gasfraktionen ein oder mehrere Behälter und/oder eine oder mehrere Leitungen mit einem Berstdruck vorgesehen sind, der zumindest dem Zehnfachen des Betriebsdruckniveaus entspricht.
